Europäisches Patentamt

(19) European Patent Office

Office européen des brevets

(11) Publication number: 0 142 222
A1

## (12) EUROPEAN PATENT APPLICATION

(21) Application number: 84305591.4

(22) Date of filing: 17.08.84

(51) Int. Cl.⁴: A 61 M 25/00
A 61 M 5/14

(30) Priority: 19.08.83 US 524727

(43) Date of publication of application:
22.05.85 Bulletin 85/21

(84) Designated Contracting States:
DE FR GB

(71) Applicant: WARNER-LAMBERT COMPANY
201 Tabor Road
Morris Plains New Jersey 07950(US)

(72) Inventor: Cartmell, Robert Louis
2905 California Avenue
Kettering Ohio 45419(US)

(72) Inventor: Daugherty, Charles William
1726 Paintersville New Jasper Road
Xenia Ohio 45385(US)

(72) Inventor: Ireland, David Bruce
6980 Hemple Road
Dayton Ohio 45418(US)

(74) Representative: Jones, Michael Raymond et al,
HASELTINE LAKE & CO. Hazlitt House 28 Southampton
Buildings Chancery Lane
London WC2A 1AT(GB)

(54) Infusion hub and tubing assembly.

(57) A hub and tubing assembly (100) suitable for use in intravenous infusion is disclosed, which assembly (100) is formed as an integral unit from polyurethane and comprises:
a length of flexible polyurethane tubing (104); and
a fluid-receiving hub (102) having a first end region (110) adapted to be connected to a source of infusion fluid, a second end region (118) adapted to receive one end of the flexible polyurethane tubing, and a passageway (116, 118) for allowing infusion fluid to flow to the tubing (104);
wherein hub (102) and tubing (104) are bonded into an integral unit (100) by means of radio frequency heating. The assembly is not difficult to produce, and can be made leak-proof in the region of the bond.

FIG-2

EP 0 142 222 A1

0142222

INFUSION HUB AND TUBING ASSEMBLY

This invention relates generally to intravenous infusion and, more particularly, to an infusion hub and tubing assembly for intravenous infusion which is assembled from a plurality of polyurethane parts and formed into an integral unit by means of dielectric heating using radio frequency energy.

Commonly used intravenous infusion assemblies comprise needle and catheter sets wherein a needle extends within a flexible catheter. The catheter assembly is connected to the infusion source by a length of catheter tubing which is joined to a hub where the connection is made.

Once positioned, the needle is removed from the catheter leaving only the flexible catheter in place such that the vein is not likely to be ruptured as a result of minor relative movements between the infusion assembly and the vein. Two varieties of combination needle/catheter sets are shown respectively in U.S. Patent No. 3,094,122 issued January 18, 1963 to Gauthier et al. and U.S. Patent No. 4,362,156 issued December 7, 1982 to Feller et al.

It is important that the individual elements forming the infusion assembly be securely bonded together to prevent leakage from the assembly and detachment of the catheter. Many of the solutions passed through an intravenous infusion assembly may cause discomfort or pain if allowed to contact a patient's skin. This is particularly true with many chemotheraputic agents utilized to treat cancer patients. Even if the solution is not toxic, bacterial contamination is a problem once an opening appears in the intravenous infusion assembly. The complications of a detached catheter within a patient's vein are potentially even graver. Accordingly, serious problems can arise if the catheter assembly is not properly manufactured.

In the prior art, a variety of methods have been utilized to interconnect the parts making up such an assembly. The parts have been mechanically interconnected by forcing a conical wedge into the proximal end of the catheter to lock the catheter in the catheter hub. Such mechanical interconnection is often referred to as "staking" or "swedging" and can be expensive and time consuming. Furthermore, the presence of the wedge in the infusion stream creates turbulent flow which can interfere with metering the infusion liquid.

The parts have also been glued together by means of an appropriate adhesive. However, gluing requires a biocompatible adhesive and is typically messy. Gluing also can lead to inadvertent blockage of a portion of the assembly passageway particularly when small gauge catheters are utilized.

Solvent bonding has also been utilized. In solvent bonding, each of the parts is made from a material which is soluble in a particular solvent. When the solvent is applied to the parts and the parts are intermated, dissolved surface portions of the two intermingle with one another to form a bond. Unfortunately, solvent bonding has proved to be only marginally reliable in preventing leakage. Solvent bonding also is expensive and time consuming when used for the assembly of intravenous infusion devices.

One assembly means which has been used in forming a Foley catheter unit is by welding the individual parts together using dielectric heating caused by radio frequency (RF) energy. However, radio frequency welding or bonding has not been applied in the manufacture of intravenous infusion assemblies. Problems encountered in RF bonding have centered around the frequencies and power levels utilized as well as the formation of the power applying electrodes. For example, if insufficient power is applied to the parts, the welds or bonds are not reliable. On the other hand, excessive power can lead to arcing of the radio frequency energy at the electrodes and may damage the RF power supply and/or cause the small openings of the passageways through the intravenous infusion assembly to be blocked.

Thus, the need exists for an integral catheter hub and tubing assembly which will provide high reliability against leakage of possibly toxic materials onto a patient's skin as well as against detachment of the catheter tubing from the hub.

According to one aspect of the present invention, there is provided a hub and tubing assembly suitable for use in for intravenous infusion, which assembly is formed as an integral unit from polyurethane and comprises:

a length of flexible polyurethane tubing; and

a fluid-receiving hub having a first end region adapted to be connected to a source of infusion fluid, a second end region adapted to receive one end of the flexible polyurethane tubing, and a passageway for allowing infusion fluid to flow to the tubing;

wherein hub and tubing are bonded into an integral unit by means of radio frequency heating.

Another aspect of the present invention provides a method for making an integral polyurethane hub and tubing assembly suitable for use in intravenous infusion, the assembly comprising a fluid-receiving hub and a length of tubing, the hub having a first end region adapted to be connected to a source of infusion fluid, a second end region adapted to receive one end of the tubing, and a passageway for allowing infusion fluid to form from the first to the second end of the hub; which method comprises:-

inserting one end of the length of tubing into the second end region of the hub; and

applying radio frequency energy to that zone of the hub into which the tubing is inserted to heat sufficiently the hub zone and the tubing such that the polyurethane materials of the hub and the tubing flow into one another to weld the tubing into the hub.

## Summary of the Invention

In accordance with the present invention, a hub and tubing assembly for intravenous infusion is formed as an integral unit from a plurality of polyurethane parts by means of welding the parts together using dielectric heating created by radio frequency energy. It has been found that catheter assemblies bonded by radio frequency heating in accordance with the present invention are particularly advantageous because the assembly can be manufactured from parts made from polyurethanes having the same or different hardness ratings and yet these parts can be integrally bonded together such that there is intermingling of their constituent polymers and they cannot be separated.

In accordance with the present invention, a section of flexible polyurethane tubing is inserted into a polyurethane fluid receiving hub and radio frequency energy is applied to the portion of the hub into which the tubing is inserted to heat the tubing and the hub such that they melt into one another to form a reliable weld or bond. Preferably, the fluid receiving hub includes tubing stop means, for example, an internal shoulder sized to engage the end of the tubing, for defining a tubing insertion point beyond which the tubing cannot be inserted into the hub. Such assembly and welding operations using radio frequency energy provide a hub and tubing assembly which is an integral unit, i.e., the individual elements forming the infusion assembly are so bonded to one another that the polymers at the interface of the hub and tubing are intermingled.

The present invention also provides a process for making an integral polyurethane catheter hub and tubing assembly for intravenous infusion comprising the steps of interconnecting the hub and tubing together and applying the appropriate frequency and energy levels of RF energy to the section of the hub receiving the tubing such that the polyurethane compounds of the energized portions of the hub and tubing are heated such that they flow into one another to form an integral assembly.

It is, therefore, an object of the present invention to provide an improved intravenous hub and tubing assembly which is formed as an integral unit from a hub and a tubing formed from polyurethane compounds having the same or different hardness, the individual hub and tubing being assembled and welded to form an integral unit by means of the application of the appropriate frequency and energy levels of radio frequency energy to interengaging portions of the assembly to thereby fuse the interengaging portions and firmly weld them to one another.

Other objects and advantages of the invention will be apparent from the following description, the accompanying drawings and the appended claims.

### Brief Description of the Drawings

Fig. 1 is a plan view of a hub and tubing assembly in accordance with the present invention.

Fig. 2 is a sectional view of the fluid infusion hub of Fig. 1 taken along the line 2-2.

Fig. 3 is a side view of an apparatus for engaging a fluid infusion hub and applying radio frequency energy to a section of that hub into which a section of tubing has been inserted.

Fig. 4 shows an end view of electrodes for conducting radio frequency energy to the fluid infusion hub and tubing combination.

### Detailed Description of the Invention

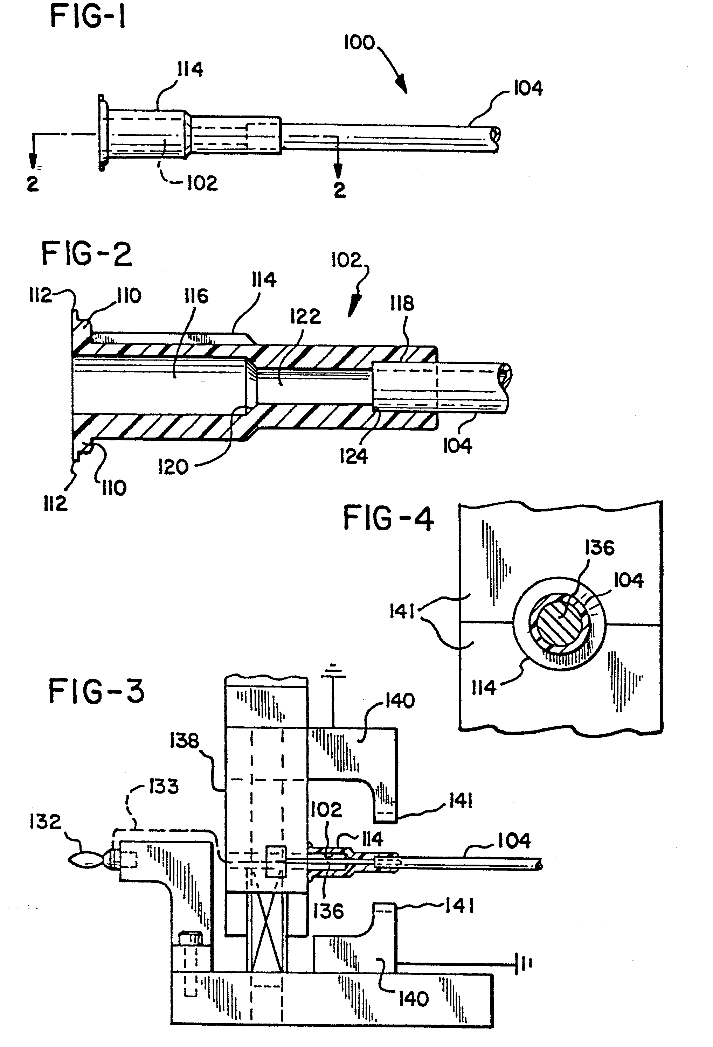

In Fig. 1, an infusion hub and tubing assembly 100 in accordance with the present invention comprises a fluid receiving hub 102, and a section of flexible tubing 104, formed into an integral unit by means of radio frequency welding or bonding as will be described hereinafter.

The fluid receiving hub 102, shown in cross-section in Fig. 2, comprises a hollow, generally cylindrical, body molded from a relatively rigid polyurethane compound. Typically, the polyurethane forming the hub has a Shore Durometer D rating of about 50 to 75 and the polyurethane forming the tubing has a Shore Durometer A hardness of about 55 to 70.

The proximal end of the hub 102 has a collar 110 with radially and circumferentially extending lugs 112 that may be used with conventional equipment employing Luer locks for external connection to a source of infusion fluid. Reinforcing ribs 114 run approximately two-thirds of the way along the hub from the collar 110 toward the distal end of the hub.

The hub 102 comprises a primary fluid receiving cavity 116 opening to its proximal end and a tubing receiving canal 118 opening to the distal end of the hub 102. The primary fluid receiving cavity 116 and the tubing receiving canal 118 are interconnected by a diameter reducing frustoconical section 120 and a generally cylindrical interconnecting canal 122 which is of a smaller diameter than the diameter of the generally cylindrical tubing receiving canal 118. The reduced diameter canal 122 forms a tubing stop means or shoulder 124 such that when the tubing 104 is inserted into the tubing receiving canal 118, it is extended only until it engages the shoulder 124 to provide a selected or defined length of the tubing 104 extending into the hub 102.

Fig. 3 shows a jig for welding the tubing 104 to the hub 102. RF energy from a Solidyne KH 8 RF generator from Solidyne, Inc. (not shown) is connected to terminal lugs 132 of the jig shown in Fig. 3 which in turn is connected to a metal pin 136 on which the hub and tubing assembly is mounted as it is welded. An alternating current is delivered to the pin 136 by the generator which causes the polarity of the pin to rapidly oscillate between a highly positive and a highly negative polarity with respect to ground. Thus, an alternating electric field is set up between the pin 136 and the electrodes 140 which are grounded.

Because it is difficult to maintain uniform voltage distribution over large areas and a relatively small bond area is sufficient to form the assembly into an integral unit, electrodes 140 are designed with smaller dimension tips 141 where the field is concentrated. The portions of the catheter hub and tubing assembly in the alternating field between the electrode tips 141 heat in a known manner. It is not essential that there be a space (Fig. 4) between the catheter assembly 100 and the ground electrodes 140 to prevent arcing. Sharp points and edges are avoided in the electrodes wherever possible since these points are the first places that breakdown occurs.

For welding the tubing 104 to the hub 102, a generally cylindrical pin 136 extends from the framework 138 of the jig shown in Fig. 3 with the generally cylindrical pin 136 being connected to the RF generator. The ground electrodes 140, an end view of which is shown in Fig. 4, are closed to engage one another and form an intimate contact from the forward portion of the hub 102 into which the tubing 104 has been inserted.

Bonding conditions will vary depending on the characteristics of the specific polyurethanes used, the electrode construction and the surroundings. The maximum voltage that can be used is limited by the voltage breakdown characteristics.

As a general rule, the frequency should be as high as possible so that the lowest voltage can be employed. However, at higher frequencies the equipment is more costly, it is difficult to deliver the power to the material as efficiently and it is more difficult to maintain uniform voltage distribution.

It has been found that RF energy at 1 to 100 mHz, preferably 25 to 70 mHz and most preferably 55 to 65 mHz is preferred for bonding the polyurethane parts of the hub and tubing assembly of the present invention. However, there may be small changes in the preferred frequency as the polyurethane compositions change.

For bonding, the RF energy is connected at a power level of about 10 to 5,000 watts for a period of approximately 0.1 to 60 seconds, preferably 100 to 300 watts for 2 to 11 seconds, and actually at 200 watts for 4 seconds. It should be understood that the period can vary from 3 to 7 seconds depending upon the specific composition of the polyurethane compounds making up the tubing 104 and the hub 102 as well as the specific size and thickness of the hub and tubing.

The RF field is maintained for a period sufficient to melt the parts of the assembly only in the vicinity of the desired bond such that the polyurethanes forming the hub and tubing mingle but without loosing their form or blocking the channel through the assembly. The use of the electrode pin 136 makes the upper limits of the RF power applied to weld the tubing 104 to the hub 102 somewhat less restrictive, since the pin 136 maintains the opening into the tubing 104 in spite of marginal excesses of heating generated by the RF energy passing through the polyurethane materials.

CLAIMS:

1. A hub and tubing assembly suitable for use in    intravenous infusion, which assembly is formed as an integral unit from polyurethane and comprises:

a length of flexible polyurethane tubing; and

a fluid-receiving hub having a first end region adapted to be connected to a source of infusion fluid, a second end region adapted to receive one end of the flexible polyurethane tubing, and a passageway for allowing infusion fluid to flow to the tubing;

wherein hub and tubing are bonded into an integral unit by means of radio frequency heating.

2. An assembly as claimed in Claim 1, wherein the second end region of the hub includes a tubing stop means for defining a tubing insertion point beyond which the tubing cannot be inserted into the hub.

3. An assembly as claimed in Claim 2, wherein the tubing stop means comprises an internal shoulder sized to engage the end of the tubing as it is inserted into the hub to define the tubing insertion point.

4. An assembly as claimed in Claim 1, 2 or 3, wherein the hub is formed from a harder polyurethane than the tubing.

5. A method for making an integral polyurethane hub and tubing assembly suitable for use in intravenous infusion, the assembly comprising a fluid-receiving hub and a length of tubing, the hub having a first end region adapted to be connected to a source of infusion fluid, a second end region adapted to receive one end of the tubing, and a passageway for allowing infusion fluid to flow from the first to the second end of the hub; which method comprises:-

inserting one end of the length of tubing into the second end region of the hub; and

applying radio frequency energy to that zone of the hub into which the tubing is inserted to heat sufficiently the hub zone and the tubing such that the polyurethane materials of the hub and the tubing flow into one another to weld the tubing into the hub.

6. A method according to Claim 5, wherein the radio frequency energy is applied at a frequency of from 1 to 100 mHz.

7. A method according to Claim 5 or 6, wherein the radio frequency energy is applied at a power level of from 10 to 5,000 watts and for a period of from 0.1 to 60 seconds.

8. A method according to Claim 5, 6 or 7, wherein the hub is formed from a harder polyurethane than the tubing.

9. An integral polyurethane catheter hub and tubing assembly formed in accordance with a method according to any one of Claims 5 to 8.

FIG-1

FIG-2

FIG-4

FIG-3

**EUROPEAN SEARCH REPORT**

| | DOCUMENTS CONSIDERED TO BE RELEVANT | | EP 84305591.4 |
|---|---|---|---|

| Category | Citation of document with indication, where appropriate, of relevant passages | Relevant to claim | CLASSIFICATION OF THE APPLICATION (Int. Cl.4) |
|---|---|---|---|
| Y | US - A - 3 769 975 (M. NIMOY) | 1 | A 61 M 25/00 |
| A | * Totality; especially column 2, lines 6-44 * | 4,5,8 | A 61 M 5/14 |
| | -- | | |
| Y | EP - A1 - 0 040 508 (SHILEY CORP.) | 1 | |
| A | * Totality; especially page 10, lines 19-26; page 19, lines 34,35 * | 5,6,7 | |
| | -- | | |
| A | US - A - 4 192 305 (CH.H. SEBERG) | 1,2,4, 5,8 | |
| | * Totality; especially fig. 1,12; column 3, line 31 - column 4, line 5 * | | |
| | -- | | |
| A | US - A - 4 177 809 (H.R. MOOREHEAD) | 1,4,5, 8 | |
| | * Totality; especially fig.1,3; column 4, lines 22-68 * | | TECHNICAL FIELDS SEARCHED (Int. Cl.4) |
| | ---- | | A 61 M 5/00 |
| | | | A 61 M 25/00 |
| | | | B 29 C 27/00 |

The present search report has been drawn up for all claims

| Place of search | Date of completion of the search | Examiner |
|---|---|---|
| VIENNA | 30-01-1985 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82